# EUROPEAN PATENT SPECIFICATION

(11) **EP 0 722 305 B1**
(45) Date of publication and mention of the grant of the patent: **16.12.1998**
(21) Application number: 94928656.1
(22) Date of filing: 23.09.1994
(51) Int. Cl.: A61F 13/15

(54) **ABSORBENT ARTICLE HAVING ELASTIC PLEAT**
SAUGFÄHIGER GEGENSTAND MIT ELASTISCHER FALTE
ARTICLE ABSORBANT COMPORTANT UNE PARTIE PLISSEE ELASTIQUE

(30) Priority: 29.09.1993 US 129534
(43) Date of publication of application: 24.07.1996
(73) Proprietor: THE PROCTER & GAMBLE COMPANY, Cincinnati, Ohio 45202 (US)
(72) Inventor: TAYLOR, JoAnn, Lee, Trenton, OH 45067 (US)
(74) Representative: Casey, Lindsay Joseph
(86) International application number: US9410809
(87) International publication number: WO9508972

(56) References cited:
- EP-A- 0 534 488
- WO-A-92/14430
- WO-A-93/10733
- US-E- 33 106

## Description

### BACKGROUND OF THE INVENTION

### 1. Technical Field

The present invention relates generally to disposable absorbent articles and, more particularly, to the incorporation of an elasticized pleat into the side flaps of a bordered catamenial pad.

### 2. Background Art

Disposable absorbent articles are commercially available in a wide variety of configurations for a variety of purposes, including absorbing and retaining discharged body fluids. For example, disposable diapers and incontinent briefs are intended to absorb and contain urine and feces, bandages are intended to absorb and contain blood and other body exudates, while catamenial pads are intended to absorb and retain menstrual fluids and other vaginal discharges. Regardless of the specific application, each absorbent article is intended to isolate the discharged fluids for preventing the undergarments, clothing, bedding etc. surrounding the disposable article from becoming soiled.

As is generally known, the basic structure for most disposable absorbent articles includes an absorbent core that is encased between a liquid permeable topsheet and a liquid impermeable backsheet. In addition, it is also common to extend the topsheet and backsheet beyond the periphery of the absorbent core for providing a continuous and relatively flexible border segment. In an effort to inhibit the lateral migration or leakage of fluids from the absorbent core, some absorbent articles incorporate a liquid impervious "barrier" mechanism into the border segment. As will be appreciated, such leakage is undesirable because it may cause soiling of the wearer's undergarments and/or clothing in the vicinity surrounding the absorbent article.

One method of inhibiting such leakage is to incorporate a liquid impervious seal into the peripheral border segment of the disposable absorbent article. For example, U.S. Pat. No. 4,321,924 to Ahr, entitled "Bordered Disposable Absorbent Article", discloses the incorporation of a pair of liquid impermeable seams into the channeled border of a catamenial pad. Alternatively, it is also known to incorporate an elastic component into the longitudinal side margins of the peripheral border segment for providing elasticized side barriers. Disposable absorbent articles of this class are generally disclosed in U.S. Pat. No. 4,758,241 to Papajohn which is entitled "Menstrual And Incontinence Pad", and U.S. Pat. No. 5,032,121 to Mokry which is entitled "Absorbent Article Having A Cup-Shaped Configuration". However, such conventional articles have gathered or "ruffled" elasticised side flaps which result in numerous gaps, rather than a smooth seam for a better fit. Moreover, such elasticized side flaps have typically been used in relatively bulky absorbent articles.

European Patent Specification No. EP-A-0,534,488 discloses an absorbent article having gaskets for preventing lateral leakage of fluid. Each of the gaskets comprises a portion of a first layer adjacent one of its edges, and a portion of a second layer adjacent one of its edges joined to the portion of the first layer so as to form a flange. A strip of material enclosing at least a portion of the flange is also provided.

Thus, while such conventional disposable absorbent articles are generally acceptable for their intended purpose, a continuing need exists to develop improvements in the structure, method of manufacture, and cost of bordered disposable absorbent articles.

It is therefore an object of the present invention to provide a bordered disposable absorbent article having elasticized pleats incorporated into the side margins of its peripheral border segment for inhibiting the lateral migration of fluids retained within the absorbent article.

It is another object of the present invention to provide a disposable catamenial pad having an elasticized side pleat, a portion of which stands up to form a liquid impermeable sidewall that acts as a lateral barrier to the flow of menstrual fluids and other bodily exudates.

Yet another object of the present invention is to incorporate a low tension elasticized pleat into the side flaps of a bordered catamenial pad for curving the pad about a lateral axis to promote enhanced comfort and fit.

These and various other objects, advantages and features of the present invention will become apparent from the following description and claims, when considered in conjunction with the appended drawings.

### SUMMARY OF THE INVENTION

According to the present invention, a disposable absorbent article is provided having an absorbent core disposed between a liquid impermeable backsheet and a liquid permeable topsheet. The topsheet and backsheet each extend outwardly beyond the peripheral edge of the absorbent core to form a continuous border segment. In addition, the absorbent article has a pair of longitudinally extending elasticized pleats formed from side margin extensions of the border segment. The elasticized pleats have a first fold defining a first flap and a second fold defining a second flap with the ends of each flap being affixed together and/or to the topsheet. The second flap is elasticized such that contraction thereof causes the opposite ends of the second flap to move toward each other while pulling an intermediate portion of the pleat transversely away from the remainder of the article. Thus, a "stand-up" side barrier is formed which acts as a liquid impervious barrier for preventing fluid from leaking laterally from the absorbent article.

### BRIEF DESCRIPTION OF THE DRAWINGS

While the specification concludes with claims particularly pointing out and distinctively claiming the subject matter which is regarded as forming the present invention, it is believed that the invention will be better understood from the following description, taken in conjunction with the accompanying drawings in which:
Figure 1 is a perspective view of an absorbent article incorporating the novel principles of the present invention;
Figure 2 is a side view of the absorbent article shown in Figure 1;
Figure 3 is an enlarged partial cross-sectional view of a portion of the absorbent article of Figures 1 and 2, shown in a flattened condition;
Figure 4 is an enlarged partial cross-sectional view of a portion of the absorbent article taken along line 4-4 in Figure 1;
Figure 5 is an enlarged partial cross-sectional view of a portion of an alternative embodiment of the present invention;
Figure 6 is an enlarged partial cross-sectional view of another alternative embodiment of the present invention;
Figure 7 is a top plan view of yet another alternative embodiment of the present invention, in a flattened condition;
Figure 8 is a side view of the absorbent article shown in Figure 7; and
Figure 9 is a top plan view of an additional alternative embodiment of the present invention, shown in a flattened condition.

### DETAILED DESCRIPTION OF THE PREFERRED EMBODIMENTS

The present invention relates to disposable absorbent articles and, in particular, to bordered absorbent articles having elasticized side pleats which act as a barrier against lateral migration and/or leakage of body exudates. As used herein, the term "disposable absorbent article" refers to articles for absorbing and retaining liquids, such as those discharged from the human body, which articles are intended to be discarded after a single use. Accordingly, the various preferred embodiments of the disposable absorbent article of the present invention are directed to the incorporation of such elasticized side pleats into the side margins of a bordered catamenial pad. A "catamenial pad" is an absorbent article worn by females externally to the urogenital region for absorbing and containing menstrual fluids and other vaginal discharges. However, it should be understood that the following description of the various preferred embodiments of the bordered catamenial pad is merely exemplary in nature, and is in no way intended to limit the invention, its scope or applications. Likewise, it should be noted that the novel principles and objectives of the present invention may also be utilized in other disposable absorbent articles, such as diapers, panty liners, menstrual discharge pads, incontinence pads, and the like.

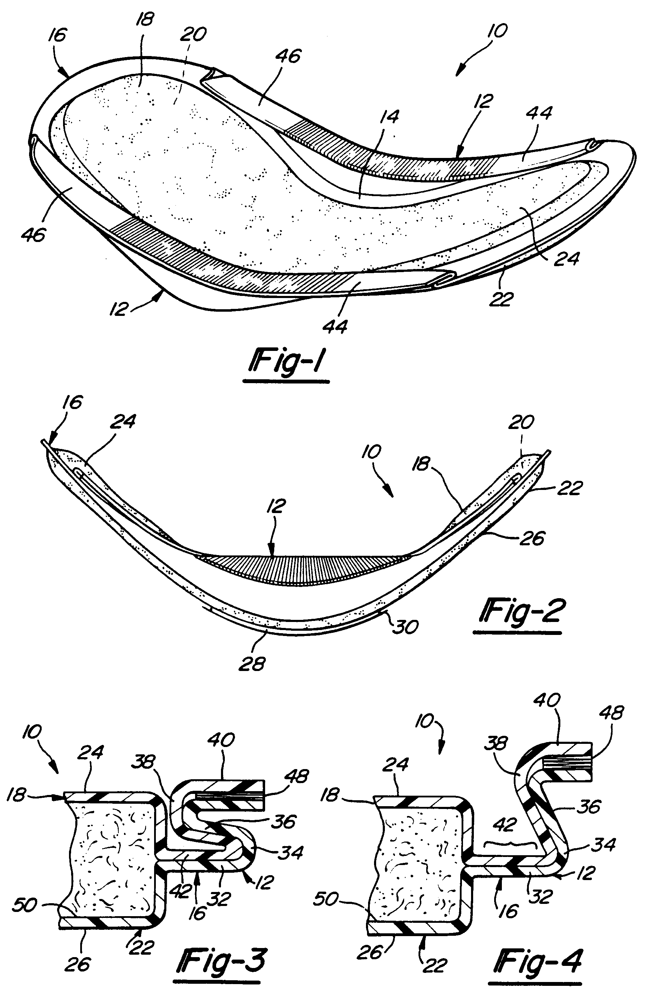

With particular reference to Figures 1 through 4, a preferred construction for a catamenial pad 10 is shown to include a pair of elasticized pleats 12 formed in the side margins 14 of its peripheral border segment 16. More specifically, catamenial pad 10 is constructed of a liquid permeable topsheet 18, an absorbent core 20, and a liquid impermeable backsheet 22. Topsheet 18 and backsheet 22 both extend outwardly beyond an outer peripheral edge of absorbent core 20 to define the continuous and relatively flexible border segment 16.

As shown in Figures 3 and 4, absorbent core 20 is disposed between topsheet 18 and backsheet 22, and may be formed of a variety of absorbent materials. The material selected for absorbent core 20 should be flexible, compressible, comfortable and non-irritating to the wearer. Moreover, the absorbent capacity of the material used in absorbent core 20 must be sufficient to absorb and retain the expected liquid loading on catamenial pad 10. Absorbent core 20 may be manufactured in a variety of shapes and sizes to comfortably fit the urogenital region of the female wearer. In addition, absorbent core 20 may be manufactured from a wide variety of liquid absorbent materials commonly used in disposable sanitary napkins, diapers and other absorbent articles, such as comminuted wood pulp which is generally referred to as airfelt, creped cellulose wadding, absorbent foams, absorbent sponges, super absorbent polymers, absorbent hydrogel materials, polymeric fibers, or any equivalent materials or combinations of materials. Preferably, absorbent core 20 includes a predetermined amount of superabsorbent polymeric material (or absorbent gelling material) particles.

Topsheet 18 is liquid permeable and has an upper or outer liquid receiving surface 24 which contacts the skin of the wearer of catamenial pad 10. Topsheet 18 should therefore be compliant, soft, and non-irritating to the skin of the wearer. Further, topsheet 18 is liquid pervious for permitting liquids to readily penetrate and transfer through its thickness. A suitable topsheet 18 may be manufactured from a wide range of materials such as formed thermoplastic films, apertured plastic films, porous foams, reticulated foams, natural fibers (i.e., wood or cotton fibers), synthetic fibers (i.e., polyester or polypropylene fibers) or from a combination of natural and synthetic fibers, with formed films being preferred. Formed films are preferred for topsheet 18 because they are pervious to liquids and yet non-absorbent. Thus, liquid receiving surface 24, which is in contact with the body, remains dry for reducing body soiling and creating a more comfortable feel for the wearer. In a preferred embodiment of the present invention, body surface 24 of topsheet 18 is hydrophilic for assisting in transferring liquid through topsheet 18. This diminishes the likelihood that menstrual fluid will flow off topsheet 18 rather than being absorbed by absorbent core 20. The term "hydrophilic" is used herein to refer to a surface which is wetted by contact with a liquid. The wetting of materials is generally defined in terms of contact angle and surface tension. These properties are discussed further in the publication of the America Chemical Society entitled "Contact Angle, Wettability, and Adhesion", edited by Robert F. Gould, copyright 1964. Topsheet 18 preferably has a multiplicity of transversely extending capillaries for encouraging liquid to rapidly penetrate or strike therethrough for subsequent absorption and retention by absorbent core 20. Topsheet 18 and backsheet 22 each have a shape similar to but larger than absorbent core 20.

Liquid impermeable backsheet 22 prevents liquid which has penetrated topsheet 18 and been absorbed by absorbent core 20 from soiling the undergarments of the wearer of catamenial pad 10. Backsheet 22 is preferably formed of a hydrophobic polyethylene film. The term "hydrophobic" is used herein to refer to a surface which is not wetted by contact with a liquid. Figure 2 also shows that a lower or outer garment surface 26 of catamenial pad 10 is provided with a fastener, such as a pressure sensitive adhesive fastener 28, for attaching catamenial pad 10 to the wearer's panties. Preferably, adhesive fastener 28 is covered with a release paper 30 to keep the adhesives from sticking to surfaces other than the panties prior to use of catamenial pad 10.

In accordance with the novel principles and features of the present invention, catamenial pad 10 incorporates a pair of longitudinally extending side pleats 12 that are formed in side margins 14 of border segment 16. Side pleats 12 of course may be formed having any suitable length and width. As best seen from the partial cross-sectional views of Figures 3 and 4, each side pleat 12 is formed from a side extension 32 provided in the side margin portion of backsheet 22, and includes a first longitudinal fold 34 defining a first flap 36 and a second longitudinal fold 38 defining a second flap 40. First flap 36 and second flap 40 are generally sinusoidal and thus overlap for covering a portion 42 of topsheet 18 and first flap 34, respectively. Side pleats 12 thus preferably assume generally a "Z" shape in cross-section, as shown in Figures 3 and 4. More particularly, side extensions 32 are first folded over topsheet 18 toward absorbent core 20 and then folded back toward an outer edge of pad 10 to form side pleats 12. Side extensions 32 are preferably formed integrally with backsheet 22, or they may be separate pieces of material which are bonded to backsheet 22.

Side pleats 12 each have a first end portion 44 and a second end portion 46 which are affixed to a portion of border segment 16 by a suitable bonding technique. The types of various bonds among the elements of catamenial pad 10 may use any of several techniques, including an adhesive, ultrasonic bonding, or heat sealing. If desired, side pleats 12 may be formed with the entire length of first and second end portions 44 and 46 bonded, or only an outer partial length of end portions 44 and 46 may be bonded. The bonds of first and second end portions 44 and 46 are preferably made by the simultaneous application of transverse pressure, possibly with heat, via a process commonly referred to as a "crimping" operation. More specifically, an upper and lower crimp tool (not shown) concurrently compress topsheet 18 and backsheet 22 in close proximity to end portions 44 and 46 of side pleats 12. The tools should be wide enough so as not to cut, mar or tear topsheet 18 or backsheet 22. During the "crimping" process, sufficient heat may be applied to the crimping tools to melt topsheet 18 and backsheet 22, thereby forming a solid yet flexible bond. However, it will be appreciated that any other suitable technique for forming a bond or seam is within the contemplated scope of the present invention.

With continued reference to Figures 1 through 4, side pleats 12 are shown provided with an elastic member 48. Elastic member 48 is applied to second flap 40 in a longitudinally stretched (i.e., tensioned) condition when catamenial pad 10 and second flap 40 are in a flattened condition. In addition, elastic member 48 extends longitudinally between first end portion 44 and second end portion 46 of second flap 40. When catamenial pad 10 and elastic member 48 are allowed to reach an equilibrium state, elastic member 48 contracts, which tends to pull first end portion 44 and second end portion 46 of pleats 12 toward each other. As a result, catamenial pad 10 is curved or bent about a lateral axis, and an intermediate portion of first flap 36 is urged upwardly relative to the remainder of pad 10. Second flap 40 preferably remains aligned approximately parallel to a corresponding portion of border segment 16. This alignment between second flap 40 and border 16, and the preferred "Z" shape, are more clearly shown in Figures 3 and 4.

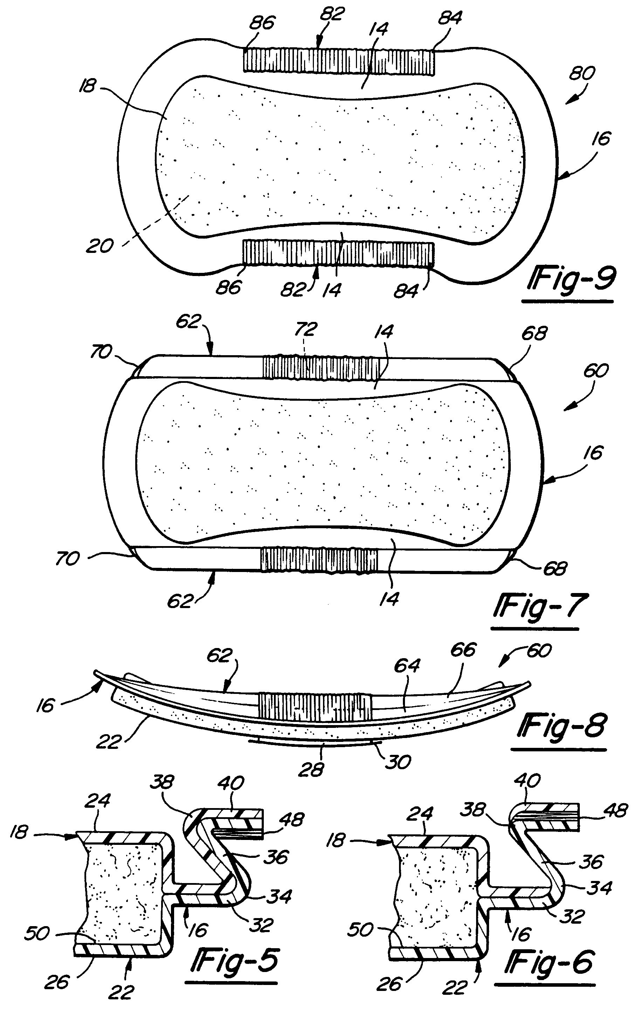

Elastic member 48 is preferably laminated between top sheet 18 and backsheet 22, as shown in Figures 3 and 4. In the alternative, elastic member 48 can be bonded to lower surface 26 of backsheet 22, as shown in Figure 5. Another alternative embodiment is shown in Figure 6, in which elastic member 48 is bonded to an upper surface 50 of backsheet 22. In the embodiment of Figure 6, topsheet 18 preferably extends outward only to first fold 34.

In addition, catamenial pad 10 assumes a cupped-shape contour similar to that shown in Figures 1 and 2, in which topsheet 18, absorbent core 20 and backsheet 22 are curved around a lateral axis. As will be appreciated, the eventual curved shape of catamenial pad 10 will depend on various physical characteristics, such as the longitudinal rigidity of pad 10, the length and width of pleats 12, and the modulus of elasticity of elastic member 48. These characteristics will affect the curvature of catamenial pad 10 and the extent of transverse extension (i.e., "stand-up") of second flaps 40. First flap 36 tends to elastically extend in a transverse direction from the remainder of pad 10 to form a sidewall, preferably defining a acute angle with respect to side margin 14, for preventing lateral leakage of fluids captured by pad 10. Thus, elasticised pleats 12 are provided for elastically hugging the wearer's body to result in more comfort and effective liquid containment.

Another alternative embodiment of the present invention is shown in Figures 7 and 8, which depict a catamenial pad 60 that is generally similar to catamenial pad 10 of Figures 1 through 4. For purposes of clarity, like reference numerals designate those elements of alternative embodiments that are similar or identical to corresponding elements of catamenial pad 10. As with the embodiment shown in Figures 1 through 4, catamenial pad 60 has a topsheet 18 and a backsheet 22 surrounding an absorbent core 20. However, catamenial pad 60 has a pair of side pleats 62 which extend for substantially the entire longitudinal length of pad 60. Pleats 62 have a first longitudinal fold defining a first flap 64 and a second longitudinal fold defining a second flap 66. Moreover, side pleats 62 each have a first end 68 and a second end 70 which are both bonded in a similar manner as end portions 40 and 42 of catamenial pad 10. According to this construction, an elastic member 72 is provided in a central region of second flap 66. In Figure 7, catamenial pad 60 is shown in a flattened condition in which elastic member 72 is applied and affixed to second flap 66 in a tensioned condition. When pad 60 is allowed to reach a equilibrium position, pad 60 tends to assume a curved cup-like shape similar to that shown in Figure 8. As such, second flap 66 tends to contract for pulling ends 68 and 70 toward each other, whereby first and second flaps 64 and 66 extend transversely from first flap 64 to form the long upstanding sidewalls shown. The longer sidewalls of catamenial pad 60 may thus provide more effective liquid containment and comfort.

Yet another alternative embodiment of the present invention is illustrated in Figure 9, which shows a catamenial pad 80 which is generally similar to pad 10 of Figures 1 through 4. Catamenial pad 80 provides a pair of pleats 82 formed generally similar to pleats 12 which extend longitudinally for only a portion of the length of pad 80. Pleats 82 have a first and second end 84 and 86 which are bonded to a portion of border segment 16.

## Claims

1. A disposable absorbent article (10) having a lateral centerline and a longitudinal length, said absorbent article (20) comprising:
a liquid permeable topsheet (18);
a liquid impermeable backsheet (22) joined with said topsheet (18);
an absorbent core (20) disposed between said topsheet (18) and said backsheet (22), and defining a perimeter; and wherein said topsheet (18) and said backsheet (22) extend outwardly beyond the perimeter of said absorbent core (20) to form a peripheral border segment (16) which defines a longitudinally extending side margin (14);
said longitudinally extending side margin (14) being characterized by an elongated pleated portion (12), said pleated portion (12) comprising:
a first flap (36) formed by a first fold (34) in said extension of said topsheet (18) and/or backsheet (22),
a second flap (40) formed by a second fold (38) in said extension of said backsheet (22) and/or topsheet (18); and
an elastic member (48) extending longitudinally on both sides of said lateral centerline and affixed to said second flap (40) which elasticizes said second flap (40) and induces said absorbent article (10) to bend about a lateral axis and for forming said pleated portion (12) into a transversely extending side barrier.

2. An article (10) as claimed in Claim 1 wherein the longitudinal length of said elastic member (48) defines the length of said pleated portion (12) and said pleated portion (12) has a first end (44) and a second end (46) which are affixed to underlying portions of said absorbent article (10) so that said elastic member (48) pulls a portion of said first flap (36) away from a portion of said border segment and forms said transversely extending side barrier.

3. An article (10) as claimed in Claim 1 or Claim 2 wherein said pleated portion (12) extends for substantially the longitudinal length of said article (10).

4. An article as claimed in any of Claims 1-3 wherein a central portion of said second flap (40) is elasticized.

5. An article as claimed in any of Claims 1-4 wherein said elastic member (48) further comprises a strip of elastic material.

6. An article as claimed in Claim 5 wherein said elastic strip (48) is affixed to said second flap (40) on one of an upper or a lower surface or between said topsheet (18) and said backsheet (22).

7. An article as claimed in any of Claims 1-6 wherein said pleated portion (12) is formed from an extended portion of said backsheet (22) which extends outwardly beyond a perimeter defined by said border segment.

8. An article as claimed in any of Claims 1-7 wherein said elastic member (48) is adapted to cause said article (10) to be biased into a cup-like shape.

9. An article as claimed in any of Claims 1-8 wherein said transversely extending side barrier (14) is also upstanding.

10. An article as claimed in any of Claims 1-9 wherein said side barrier (14) is liquid impervious.

## Patentansprüche

1. Ein wegwerfbarer absorbierender Artikel (10) mit einer Quermittellinie und einer der Länge nach verlaufenden Länge, wobei der genannte absorbierende Artikel (20) umfaßt:
ein flüssigkeitsdurchlässiges Deckblatt (18);
ein flüssigkeitsundurchlässiges Rückenblatt (22), welches mit dem genannten Deckblatt (18) verbunden ist;
einen absorbierenden Kern (20), welcher zwischen dem genannten Deckblatt (18) und dem genannten Rückenblatt (22) angeordnet ist und einen Umfang definiert; und wobei das genannte Deckblatt (18) und das genannte Rückenblatt (22) sich über den Umfang des genannten absorbierenden Kerns (20) hinaus auswärts erstrecken, um ein peripheres Einfassungssegment (16) zu bilden, welches einen sich der Länge nach erstreckenden Seitenrandstreifen (14) definiert;
wobei der genannte sich der Länge nach erstreckende Seitenrandstreifen (14) gekennzeichnet ist durch einen länglichen gefältelten Abschnitt (12), wobei der genannte gefältelte Abschnitt (12) umfaßt:
einen ersten Lappen (36), welcher durch eine erste Faltung (34) in der genannten Verlängerung des genannten Deckblatts (18) und/oder des genannten Rückenblatts (22) gebildet ist,
einen zweiten Lappen (40), welcher durch eine zweite Faltung (38) in der genannten Verlängerung des genannten Rückenblatts (22) und/oder des genannten Deckblatts (18) gebildet ist; und
einen elastischen Bauteil (48), welcher sich der Länge nach an beiden Seiten der genannten Quermittellinie erstreckt und am genannten zweiten Lappen (40) fixiert ist, welcher den genannten zweiten Lappen (40) elastifiziert und den genannten absorbierenden Artikel (10) induziert, sich um eine Querachse zu biegen und um den genannten gefältelten Abschnitts (12) zu einer sich quer erstreckenden Seitenbarriere auszubilden.

2. Ein Artikel (19), wie er in Anspruch 1 beansprucht ist, bei welchem die sich der Länge nach erstreckende Länge des genannten elastischen Bauteils (48) die Länge des genannten gefältelten Abschnitts (12) definiert und der genannte gefältelte Abschnitt (12) ein erstes Ende (44) und ein zweites Ende (46) aufweist, welche an darunterliegenden Abschnitten des genannten absorbierenden Artikels (10) fixiert sind, sodaß der genannte elastische Bauteil (48) einen Abschnitt des genannten ersten Lappens (36) von einem Abschnitt des genannten Einfassungssegments weg zieht und die genannte sich quer erstreckende Seitenbarriere bildet.

3. Ein Artikel (10), wie er in Anspruch 1 oder Anspruch 2 beansprucht ist, bei welchem der genannte gefältelte Abschnitt (12) sich über im wesentlichen die sich der Länge nach erstreckende Länge des genannten Artikels (10) erstreckt.

4. Ein Artikel, wie er in einem der Ansprüche 1 bis 3 beansprucht ist, bei welchem ein mittlerer Abschnitt des genannten zweiten Lappens (40) elastifiziert ist.

5. Ein Artikel, wie er in einem der Ansprüche 1 bis 4 beansprucht ist, wobei der genannte elastische Bauteil (48) weiters einen Streifen elastischen Materials umfaßt.

6. Ein Artikel, wie er in Anspruch 5 beansprucht ist, bei welchem der genannte elastische Streifen (48) am genannten zweiten Lappen (40) an einer von einer oberen oder einer unteren Oberfläche oder zwischen dem genannten Deckblatt (18) und dem genannten Rückenblatt (22) fixiert ist.

7. Ein Artikel, wie er in einem der Ansprüche 1 bis 6 beansprucht ist, bei welchem der genannte gefältelte Abschnitt (12) aus einem verlängerten Abschnitt des genannten Rückenblatts (22) gebildet ist, welcher sich auswärts über einen Umfang, welcher vom genannten Einfassungssegment definiert ist, hinaus erstreckt.

8. Ein Artikel, wie er in einem der Ansprüche 1 bis 7 beansprucht ist, bei welchem der genannte elastische Bauteil (48) adaptiert ist, um den genannten Artikel (10) zu veranlassen, zu einer napfartigen Gestalt hochgezogen zu werden.

9. Ein Artikel, wie er in einem der Ansprüche 1 bis 8 beansprucht ist, bei welchem die genannte sich quer erstreckende Seitenbarriere (14) ebenso nach oben steht.

10. Ein Artikel, wie er in einem der Ansprüche 1 bis 9 beansprucht ist, bei welchem die genannte Seitenbarriere (14) flüssigkeitsundurchlässig ist.

## Revendications

1. Article absorbant jetable (10) présentant un axe central latéral et une longueur longitudinale, ledit article absorbant (20) comprenant :
une feuille de dessus perméable aux liquides (18) ;
une feuille de fond imperméable aux liquides (22) réunie à ladite feuille de dessus (18) ;
une âme absorbante (20) disposée entre ladite feuille de dessus (18) et ladite feuille de fond (22), et définissant un périmètre ; et dans lequel ladite feuille de dessus (18) et ladite feuille de fond (22) s'étendent vers l'extérieur au-delà du périmètre de ladite âme absorbante (20) pour former un segment de bordure périphérique (16) qui définit une lisière latérale s'étendant dans le sens longitudinal (14) ;
ladite lisière latérale s'étendant dans le sens longitudinal (14) étant caractérisée par une partie plissée allongée (12), ladite partie plissée (12) comprenant :
un premier rabat (36) formé par un premier pli (34) dans ledit prolongement de ladite feuille de dessus (18) et/ou de ladite feuille de fond (22),
un second rabat (40) formé par un second pli (38) dans ledit prolongement de ladite feuille de fond (22) et/ou de ladite feuille de dessus (18) ; et
un élément élastique (48) s'étendant dans le sens longitudinal sur les deux côtés dudit axe central latéral et fixé audit second rabat (40) qui élastifie ledit second rabat (40) et amène ledit article absorbant (10) à s'incurver autour d'un axe latéral et pour donner à ladite partie plissée (12) la forme d'une barrière latérale s'étendant dans le sens transversal.

2. Article (10) selon la revendication 1, dans lequel la longueur longitudinale dudit élément élastique (48) définit la longueur de ladite partie plissée (12) et ladite partie plissée (12) possède une première extrémité (44) et une seconde extrémité (46) qui sont fixées aux parties sous-jacentes dudit article absorbant (10) de manière à ce que ledit élément élastique (48) tire une partie dudit premier rabat (36) à distance d'une partie dudit segment de bordure et forme ladite barrière latérale s'étendant dans le sens transversal.

3. Article (10) selon la revendication 1 ou la revendication 2, dans lequel ladite partie plissée (12) s'étend sur sensiblement la longueur longitudinale dudit article (10).

4. Article selon l'une quelconque des revendications 1 à 3, dans lequel une partie centrale dudit second rabat (40) est élastifiée.

5. Article selon l'une quelconque des revendications 1 à 4, dans lequel ledit élément élastique (48) comprend, en outre, une bande de matériau élastique.

6. Article selon la revendication 5, dans lequel ladite bande élastique (48) est fixée audit second rabat (40) sur une des surfaces supérieure ou inférieure ou bien entre ladite feuille de dessus (18) et ladite feuille de fond (22).

7. Article selon l'une quelconque des revendications 1 à 6, dans lequel ladite partie plissée (12) est formée à partir d'une partie étendue de ladite feuille de fond (22) qui s'étend vers l'extérieur au-delà d'un périmètre défini par ledit segment de bordure.

8. Article selon l'une quelconque des revendications 1 à 7, dans lequel ledit élément élastique (48) est adapté pour amener ledit article (10) à être sollicité pour prendre une forme en coupelle.

9. Article selon l'une quelconque des revendications 1 à 8, dans lequel ladite barrière latérale s'étendant dans le sens transversal (14) est également dressée vers le haut.

10. Article selon l'une quelconque des revendications 1 à 9, dans lequel ladite barrière latérale (14) est imperméable aux liquides.
